Europäisches Patentamt

European Patent Office

Office européen des brevets

(19)

(11) Publication number: **0 022 297**
**B1**

(12) **EUROPEAN PATENT SPECIFICATION**

(45) Date of publication of patent specification: **24.08.83**

(21) Application number: **80200628.8**

(22) Date of filing: **01.07.80**

(51) Int. Cl.³: **C 07 C 15/02,**
**C 07 C 5/367,**
**C 07 C 15/073**

(54) Process for the preparation of alkyl benzenes.

(30) Priority: **07.07.79 NL 7905327**

(43) Date of publication of application:
**14.01.81 Bulletin 81/2**

(45) Publication of the grant of the patent:
**24.08.83 Bulletin 83/34**

(84) Designated Contracting States:
**AT BE DE FR GB IT NL SE**

(56) References cited:
**GB - A - 2 016 515**
**JP - A - 54 021 983**

(73) Proprietor: **STAMICARBON B.V.**
**Postbus 10**
**NL-6160 MC Geleen (NL)**

(72) Inventor: **De Graaf, Theodorus**
**Reinierstraat 2**
**NL-6191 SH Beek (L) (NL)**
Inventor: **Janssen, Ludovicus**
**Henri Hermanslaan 51**
**NL-6162 GA Geleen (NL)**

(74) Representative: **Philippens, Martin Hubert Johan**
**Jacques et al,**
**OCTROOIBUREAU DSM Postbus 9**
**NL-6160 MA Geleen (NL)**

Courier Press, Leamington Spa, England.

**0 022 297**

Process for the preparation of alkyl benzenes

The invention relates to a process for the preparation of an alkyl benzene by dehydrogenation of the corresponding alkenyl cyclohexene in the presence of a carrier-borne noble metal catalyst.

Such a process is known from Russian Patent Specification 236,462. In the process described therein, vinyl cyclohexene is converted at a temperature of 300—400°C to ethyl benzene in the presence of a catalyst consisting of palladium on carbon. A conversion of 95% and a selectivity of 92% towards ethyl benzene are hereby obtained. Such a process is also known from Journal of Catalysis 50 (1977) p. 172, in which a palladium-on-alumina catalyst is employed, the vinyl cyclohexene being completely converted to ethyl benzene.

From Japanese patent publication 54 21983 there is further known a process for producing alkylsubstituted aromatic compounds with a high selectivity by subjecting vinylcycloalkene to catalytic dehydrogenation in the gaseous phase in the presence of a catalyst consisting of Pd metal supported on an $\alpha$-type alumina carrier.

A disadvantage of these known processes is that catalyst activity decreases fairly rapidly, the catalyst being practically fully deactivated after about 1—2 weeks.

The present invention provides a process by which an alkenyl cyclohexene can be converted substantially quantitatively to the corresponding alkyl benzene with the aid of a catalyst, which even after several months of operation exhibits hardly any deterioration in activity.

According to the invention, this is achieved by using a non-acidic material as catalyst carrier and by conducting the reaction first for a minimum of 1 hour at a temperature of between 200 and 275°C and subsequently at a temperature of between 275 and 450°C.

The dehydrogenation according to the invention is performed in the presence of a noble metal catalyst. As such, platinum, palladium, ruthenium or iridium can for example be used. Palladium is preferably used as catalytically active material, because it has been found that, in contact herewith, the alkenyl cyclohexene is converted with a very high selectivity towards alkyl benzene. According to the invention, the noble metal catalyst is applied on a non-acidic carrier. For it has, surprisingly, been found that when an acidic carrier material such as alumina is used although the catalyst has a high initial activity, this activity then very rapidly decreases. As non-acidic carrier material, basic substances such as oxides, hydroxides or carbonates of calcium and/or magnesium, or barium sulphate may in the first place be used, but neutral carrier material such as carbon or neutral silicon oxide may also be used. By preference magnesium oxide is used as carrier material.

The quantity of catalyst per quantity of alkenyl cyclohexene to be converted by the process according to the invention can be varied within wide limits, for example such quantities that the space velocity, expressed as litres liquid alkenyl cyclohexene per litre catalyst per hour, is 0.01 to 500. Space velocities of between 0.5 and 50 are preferably chosen. The quantity of catalytically active noble metal in the catalyst can also be varied within wide limits, for example from 0.01—10 wt.% noble metal, calculated relative to the total catalyst weight. The quantity of noble metal in the catalyst is preferably 0.4—4 wt.%, calculated relative to the total catalyst weight. A catalyst composition of 0.5—3 wt.% palladium on magnesium oxide as carrier material has been found to be especially suitable.

In the process according to the invention the noble metal catalyst retains its high initial activity for a very long time. It has, moreover, been found that when the activity decreases, the catalyst can be regenerated in a simple manner and its original activity be restored, by passing over air or other gases containing oxygen.

According to the invention, the alkenyl cyclohexene is first passed over the catalyst for a minimum of 1 hour at a relatively low temperature of between 200 and 275°C and then at a temperature of between 275 and 450°C.

For it has been found that the catalyst is deactivated very rapidly if the alkenyl cyclohexene is brought immediately into contact with the catalyst at 275—450°C.

The alkenyl cyclohexene is preferably first passed over the catalyst for a minimum of 8 hours, more specially a minimum of 24 hours, at a temperature of between 200 and 275°C, and then at a temperature of between 275 and 450°C. The temperature preferably applied are 225—260°C and 300—350°C, respectively.

The process according to the invention is preferably performed at atmospheric pressure. Application of elevated pressure is admittedly feasible, but does not yield any extra advantages. The dehydrogenation equilibrium is moreover adversely affected by applying higher pressures.

In the process according to the invention various alkenyl cyclohexenes can be used as starting material, for example vinyl cyclohexene, isopropenyl cyclohexene, and alkyl-substituted derivatives of these. These substances can be obtained by known methods by dimerization and codimerization of conjugated diolefins. Vinyl cyclohexene, for example, can thus be obtained by dimerization of butadiene, isopropenyl cyclohexene by codimerization of butadiene and isoprene, and methyl isopropenyl cyclohexene by dimerization of isoprene.

It has been found that the presence of peroxides in the alkenyl cyclohexene to be converted affects catalyst life adversely during conversion. An alkyl cyclohexene containing less than 5 ppm

2

peroxides is therefore preferably used as starting material. Such an alkenyl cyclohexene that is substantially free of peroxides can be prepared by purifying technical-grade alkenyl cyclohexene of peroxides and storing it with substantially complete exclusion of oxygen. The removal of peroxides from alkenyl cyclohexene can be effected by known methods, for example by selective hydrogenation, dissociation by heating, or distillation over a reducing agent such as triphenyl phosphine.

Although the reaction can in principle also be carried out in the liquid phase, preference is given to carrying out the reaction in the gas phase. This reaction is preferably carried out in the presence of an inert gas, for instance nitrogen. The presence of an inert gas in the reaction mixture also has the advantage that the reaction equilibrium is favourably affected.

In principle, hydrogen can also be included in the reaction mixture, but the formation of hydrogenated byproducts such as alkyl cyclohexane then increases. The reaction is therefore preferably carried out without hydrogen being added. In the process according to the invention, the hydrogen liberated in dehydrogenation has barely any influence on the selectivity towards alkyl benzene.

In the process according to the invention, the alkenyl cyclohexene added is completely converted to a product that consists substantially quantitatively (99.5% and higher) of alkyl benzene. This product can be separated in a known way, for example cooling, from the reaction mixture formed, which also contains a quantity of hydrogen and in some cases nitrogen in addition to reaction product. The separated product can be directly applied, without further treatment, for further conversions. The dehydrogenation product of vinyl cyclohexene, for instance, which consists substantially quantitatively of ethyl benzene, can thus be directly converted to styrene. The dehydrogenation product of isopropenyl cyclohexene, viz. isopropyl benzene, can be applied as the starting material for the preparation of $\alpha$-methyl styrene, or be converted to phenol by oxidation. In the same manner, methyl isopropyl benzene, obtained from methyl isopropenyl cyclohexene, can be applied for the preparation of methyl phenol, among other compounds.

The invention is further elucidated in the following practical example and comparative examples.

Example 1

In an electrically heated glass tubular reactor with a diameter of 20 mm and a length of 50 cm, provided with a thermocouple tube, a 10-cm$^3$ catalyst bed was introduced, consisting of 3 wt.% palladium on magnesium oxide.

With complete exclusion of oxygen, a gas mixture of 4-vinyl-cyclohexene-1 and nitrogen was fed to the catalyst in a ratio of 1:1 by volume, which mixture had been obtained by evaporating liquid vinyl cyclohexene while introducing oxygen-free nitrogen. The 4-vinyl cyclohexene-1 had been previously distilled over triphenyl phosphine to remove peroxides, so that peroxides were no longer analytically demonstrable in the distilled product (<2 ppm). The gas mixture throughput rate was 1 volume liquid 4-vinylcyclohexene-1 per volume catalyst per hour. The temperature in the reactor was maintained at 250°C.

Downstream of the reactor a cooler was installed, in which the gaseous reaction mixture formed was condensed. The composition of the condensed product was periodically analyzed, and the results are given in the table below.

After 600 hours the reactor temperature was increased to 325°C and the volume ratio of 4-vinylcyclohexene-1 to nitrogen also modified to 1:3.

The gaseous reaction mixture was condensed and analyzed in the same way.

The results are again given in the table below.

The test was discontinued after a total of 1200 hours operation.

| Hours of operation | Ethyl benzene content of condensate (in %) | Remarks |
|---|---|---|
| 26 | 90 | |
| 100 | 90 | |
| 200 | 90 | |
| 314 | 89 | |
| 408 | 89 | |
| 500 | 87 | The remainder of the condensate consisted |
| 600 | 87 | substantially in toto of ethyl cyclohexane |
| 700 | 99.6 | |
| 792 | 99.5 | |
| 911 | 99.5 | |
| 1009 | 99.5 | |
| 1140 | 99.5 | |
| 1200 | 99.5 | |

It is clearly demonstrated that even after 1200 hours of operation the catalyst retains its activity.

3

**0 022 297**

Example 2 (comparative example)

In the same way as in Example 1, an identical gas mixture of 4-vinylcyclohexene-1 and nitrogen was passed through the reactor, which contained palladium on alumina (0.5 wt.% Pd) as catalyst. The reaction temperature was maintained at 250°C for 214 hours and subsequently increased to 325°C. The gaseous reaction mixture was condensed and analyzed in the same way. The results are given in the table below.

| Hours of operation | Ethyl benzene content of condensate (in %) | Remarks |
|---|---|---|
| 99 | 91 | Remainder consisted mainly of ethyl cyclohexane |
| 192 | 90 | |
| 285 | 95 | |
| 311 | 95 | |
| 314 | 95 | |
| 334 | 85 | Condensate also contained 4% unconverted 4-vinylcyclohexene-1 |

It is clearly demonstrated that even after increasing the temperature the selectivity towards ethyl benzene is considerably lower than in Example 1. In addition, both the conversion and the selectivity towards ethyl benzene show a marked decrease within a relatively short time (120 hours after temperature increase).

Example 3 (comparative example)

By the same method as in Example 1, an identical gas mixture of 4-vinylcyclohexene-1 and nitrogen was passed through the reactor, which contained the same catalyst as in Example 1. The reaction temperature was 300°C. The gaseous reaction mixture was condensed and analyzed in 20 the same way. The results are given in the table below.

| Hours of operation | Ethyl benzene content of condensate (in %) | Unconverted 4-vinyl-cyclohexene-1 (in %) |
|---|---|---|
| 28 | 97 | — |
| 77 | 97 | — |
| 100 | 96 | — |
| 116 | 94 | 0.3 |
| 142 | 85 | 3.5 |

It is clearly demonstrated that the selectivity towards ethyl benzene is lower than in Example 1. In addition, the catalyst activity decreases sharply after a relatively short time (116 hours), as regards both conversion and selectivity.

## Claims

1. Process for the preparation of an alkyl benzene by dehydrogenation of the corresponding alkenyl cyclohexene in the presence of a carrier-borne noble metal catalyst, characterized in that a non-acidic material is used as catalyst carrier, and the reaction is first conducted for a minimum of 1 hour at a temperature of between 200 and 275°C and subsequently at a temperature of between 275 and 450°C.

2. Process according to Claim 1, characterized in that the reaction is first conducted for a minimum of 8 hours at a temperature of between 200 and 275°C and subsequently at a temperature of between 275 and 450°C.

3. Process according to Claim 1 or 2, characterized in that the reaction is first conducted for a minimum of 24 hours at a temperature of between 200 and 275°C and subsequently at a temperature of between 275 and 450°C.

4. Process according to any of Claims 1—3, characterized in that the reaction is first conducted at a temperature of between 225 and 260°C and subsequently at between 300 and 350°C.

5. Process according to any of Claims 1—4, characterized in that magnesium oxide is employed as carrier material.

6. Process according to any of Claims 1—5, characterized in that the reaction is performed in the presence of a maximum of 5 ppm peroxides, calculated relative to the quantity by weight of alkenyl cyclohexene.

4

**0 022 297**

7. Process according to any of Claims 1—6, characterized in that the reaction is performed in the gas phase in the presence of an inert gas.

8. Process according to any of Claims 1—7, characterized in that the reaction is performed at atmospheric pressure.

9. Process according to any of claims 1—8 characterized in that 4-vinylcyclohexene-1 is dehydrogenated to ethylbenzene.

## Patentansprüche

1. Verfahren zur Herstellung eines Alkylbenzols durch Dehydrieren des entsprechenden Alkenylcyclohexens in Gegenwart eines Edelmetall-Trägerkatalysators, dadurch gekennzeichnet, daß ein nichtsaueres Material als Katalysatorträger verwendet wird und daß die Reaktion zunächst während mindestens 1 Stunde bei einer Temperatur zwischen 200 und 275°C und anschließend bei einer Temperatur zwischen 275 und 450°C durchgeführt wird.

2. Verfahren nach Anspruch 1, dadurch gekennzeichnet, daß die Reaktion zunächst während mindestens 8 h bei einer Temperatur zwischen 200 und 275°C und anschließend bei einer Temperatur zwischen 275 und 450°C durchgeführt wird.

3. Verfahren nach Anspruch 1 oder 2, dadurch gekennzeichnet, daß die Reaktion zunächst während mindestens 24 h bei einer Temperatur zwischen 200 und 275°C und anschließend bei einer Temperatur zwischen 275 und 450°C durchgeführt wird.

4. Verfahren nach einem der Ansprüche 1 bis 3, dadurch gekennzeichnet, daß die Reaktion zunächst bei einer Temperatur zwischen 225 und 260°C und anschließend zwischen 300 und 350°C durchgeführt wird.

5. Verfahren nach einem der Ansprüche 1 bis 4, dadurch gekennzeichnet, daß Magnesiumoxid als Trägermaterial verwendet wird.

6. Verfahren nach einem der Ansprüche 1 bis 5, dadurch gekennzeichnet, daß die Reaktion in Gegenwart von höchstens 5 ppm Peroxiden, bezogen auf die Gewichtsmenge des Alkenylcyclohexens; durchgeführt wird.

7. Verfahren nach einem der Ansprüche 1 bis 6, dadurch gekennzeichnet, daß die Reaktion in der Gasphase in Gegenwart eines Inertgases durchgeführt wird.

8. Verfahren nach einem der Ansprüche 1 bis 7, dadurch gekennzeichnet, daß die Reaktion bei Atmosphärendruck durchgeführt wird.

9. Verfahren nach einem der Ansprüche 1 bis 8, dadurch gekennzeichnet, daß 4-Vinylcyclohexen-1 zu Äthylbenzol dehydriert wird.

## Revendications

1. Procédé de préparation d'un alkylbenzène par déshydrogénation de l'alcénylcyclohexène correspondant en présence d'un catalyseur du type métal noble sur support, caractérisé en ce que l'on utilise comme support de catalyseur une matière non acide et en ce que la réaction est d'abord conduite pendant un minimum d'1 heure à une température de 200 à 275°C puis à une température de 275 à 450°C.

2. Procédé selon la revendication 1, caractérisé en ce que la réaction est d'abord conduite pendant un minimum de 8 heures à une température de 200 à 275°C puis à une température de 275 à 450°C.

3. Procédé selon la revendication 1 ou 2, caractérisé en ce que la réaction est d'abord conduite pendant un minimum de 24 heures à une température de 200 à 275°C puis à une température de 275 à 450°C.

4. Procédé selon l'une quelconque des revendications 1 à 3, caractérisé en ce que la réaction est d'abord conduite à une température de 225 à 260°C puis à une température de 300 à 350°C.

5. Procédé selon l'une quelconque des revendications 1 à 4, caractérisé en ce que l'on utilise la magnésie comme matière de support.

6. Procédé selon l'une quelconque des revendications 1 à 5, caractérisé en ce que la réaction est effectuée en présence d'un maximum de 5 ppm de peroxydes par rapport au poids de l'alcényl-cyclohexène.

7. Procédé selon l'une quelconque des revendications 1 à 6, caractérisé en ce que la réaction est effectuée en phase gazeuse en présence d'un gaz inerte.

8. Procédé selon l'une quelconque des revendications 1 à 7, caractérisé en ce que la réaction est effectuée à pression atmosphérique.

9. Procédé selon l'une quelconque des revendications 1 à 8, caractérisé en ce que l'on déshydrogène le 4-vinyl-cyclohexène-1 en éthylbenzène.